# EUROPÄISCHE PATENTANMELDUNG

(11) **EP 4 840 428 A1**
(43) Veröffentlichungstag der Anmeldung: **18.11.2026**
(21) Anmeldenummer: 26177954.0
(22) Anmeldetag: 11.05.2026
(51) Int. Cl.: A61F 9/00, B65D 47/18

(54) **VORRICHTUNG ZUR TROPFENWEISEN ABGABE EINES FLUIDS**

(30) Priorität: 15.05.2025 DE 102025118912
(71) Anmelder: Henke-Sass, Wolf GmbH, 78532 Tuttlingen (DE)
(72) Erfinder: RIEDLINGER, Axel, 72355 Schömberg (DE); RAIDT, Simon, 78573 Wurmlingen (DE); LEICHT, Marco, 72189 Vöhringen (DE)
(74) Vertreter: Patentanwälte Geyer, Fehners & Partner mbB

(57) **Zusammenfassung**

Es wird bereitgestellt eine Vorrichtung zur tropfenweise Abgabe eines Fluids, wobei die Vorrichtung (1) ein Gehäuse (2) mit einem Fluidanschluss (3), über den das abzugebende Fluid (F) zuführbar ist, eine Austrittsdüse (4), über die das Fluid tropfenweise abgebbar ist, einer Fluidverbindung (5), die den Fluidanschluss (3) mit der Austrittsdüse (4) verbindet, einer Unterseite (10) und einem Betätigungselement (6) aufweist, wobei bei Betätigung des Betätigungselements (6) eine vorbestimmte Fluidmenge zur Austrittsdüse (4) hin gefördert wird, wobei die Austrittsdüse (4) einen sich geradlinig entlang einer ersten Längsrichtung (R1) erstreckenden rohrförmigen Endabschnitt (11) mit einem abgeschrägten Abgabeende (12), das eine ebene Stirnfläche (13) umfasst, aufweist, und wobei, in einem gedachten Längsschnitt des rohrförmigen Endabschnitts (11), die ebene Stirnfläche (13) zur Unterseite (10) hin weist und mit der ersten Längsrichtung (R1) einen ersten Winkel (α) einschließt, der kleiner als 90° ist.

## Beschreibung

Die vorliegende Erfindung betrifft eine Vorrichtung zur tropfenweisen Abgabe eines Fluids.

Wenn es sich bei dem Fluid um z.B. einen Impfstoff handelt, kann mit einer solchen Vorrichtung eine tropfenweise Abgabe des Impfstoffes durchgeführt werden.

Wünschenswert ist beispielsweise eine Abgabe von Augentropfen bei Geflügeltieren zur Impfung, um somit eine okulare Impfung durchzuführen. Bisher wird eine solche okulare Impfung mit Hilfe einer Kunststoffflasche durchgeführt. Durch händischen Druck auf die Flaschenwand wird ein Tropfen an einem Auslassaufsatz erzeugt. Sobald sich der Tropfen gelöst hat, wird der Druck entfernt, so dass der restliche Impfstoff durch den entstandenen Unterdruck zurück in die Flasche gezogen wird. Tropfengröße und Tropfenanzahl sind sowohl abhängig vom Druck, der vom Anwender ausgeübt wird, als auch vom Winkel, mit dem die Flasche bei der Tropfenabgabe gehalten wird.

Bei der praktischen Anwendung zur okularen Impfung eines Tieres kann es beispielsweise vorkommen, dass sich zwei oder mehr Tropfen lösen, da in der Regel eine große Anzahl von Tieren unter hohem Zeitdruck versorgt werden müssen. Es liegt somit eine anwenderbezogene Varianz vor, die einerseits zu Schwankungen bei der Dosierung und andererseits zu einem schwer kalkulierbaren Bedarf an Impfstoff für einen quantitativ definierten Tierbestand führt.

Ferner wird durch den direkten Kontakt der Hand des Anwenders mit der Flasche eine Erwärmung des Medikaments hervorgerufen, die die Impfwirkung beeinträchtigen kann.

Ausgehend hiervon soll eine Vorrichtung zur tropfenweisen Abgabe eines Fluids bereitgestellt werden, mit der die beschriebenen Schwierigkeiten möglichst vollständig überwunden werden können.

Die Erfindung ist im Anspruch 1 definiert. Vorteilhafte Weiterbildungen sind in den abhängigen Ansprüchen angegeben.

Es wird eine Vorrichtung zur tropfenweise Abgabe eines Fluids Vorrichtung (die auch als Tropfenapplikator und insbesondere als Tropfenapplikator zur okularen Impfung bezeichnet werden kann) bereitgestellt, wobei die Vorrichtung ein Gehäuse mit einem Fluidanschluss, über den das abzugebende Fluid zuführbar ist, eine Austrittsdüse, über die das Fluid tropfenweise abgebbar ist, einer Fluidverbindung, die den Fluidanschluss mit der Austrittsdüse verbindet, einer Unterseite und einem Betätigungselement aufweist. Bei Betätigung des Betätigungselements wird eine vorbestimmte Fluidmenge zur Austrittsdüse hin (bevorzugt zur Abgabe eines einzelnen Fluidtropfens) gefördert, wobei die Austrittsdüse einen sich geradlinig entlang einer ersten Längsrichtung erstreckenden rohrförmigen Endabschnitt mit einem abgeschrägten Abgabeende, das eine ebene (bevorzugt ringförmige) Stirnfläche umfasst, aufweist. Die ebene Stirnfläche weist in einem gedachten Längsschnitt des rohrförmigen Endabschnitts zur Unterseite hin und schließt mit der ersten Längsrichtung einen ersten Winkel ein, der kleiner als 90° ist.

Bei der bestimmungsgemäßen Verwendung kann die erfindungsgemäße Vorrichtung mit einer Hand ergonomisch gehalten (ohne dass z.B. das Handgelenk bei der Bedienung zusätzlich abgewinkelt werden muss) und bedient werden, wobei die ebene Stirnfläche des Abgabeendes zum Boden zeigen kann. Insbesondere kann die Stirnfläche parallel zur Erdoberfläche bzw. zur Horizontalen ausgerichtet sein. Dadurch kann eine hohe Dosiergenauigkeit (einschließlich einer wiederholbaren Abgabe eines einzelnen Tropfens) gewährleistet werden.

Daraus kann ferner eine verringerte Varianz der Tropfengröße resultieren, da die Adhäsionskräfte des Fluids (z.B. des Medikaments oder Impfstoffs) nur an der Stirnfläche wirken und eine Benetzung und insbesondere eine zunehmende Benetzung der Außenfläche des rohrförmigen Endabschnitts ausgeschlossen werden.

Man kann den ersten Winkel, im gedachten Längsschnitt, auch als zur Unterseite hin offen bezeichnen. Ferner ist es möglich, im gedachten Längsschnitt, das abgeschrägte Abgabeende dadurch zu charakterisieren, dass die Stirnfläche und die zur Unterseite weisende Außenfläche des Endabschnitts V-förmig angeordnet sind, wobei das V von der Unterseite weg (zur Oberseite des Gehäuses) hin öffnet.

Bei dem Fluid kann es sich um eine Flüssigkeit und/oder ein Medikament (wie z.B. einen Impfstoff) handeln. Der Tropfenapplikator kann insbesondere zur okularen Impfung eingesetzt werden und kann somit dazu geeignet sein.

Der erste Winkel kann kleiner oder gleich 85°, 80°, 75°, 70°, 65°, 60°, 55°, 50° oder 45° sein. Ferner kann der erste Winkel größer oder gleich 10°, 15°, 20°, 25°, 30°, 35°, 40°, 45°, 50°, 55°, 60°, 65°, 70°, 75°, 80° oder 85° sein. Bevorzugt weist der erste Winkel einen Wert aus dem Bereich 40° - 60° (einschließlich der Grenzen) und besonders bevorzugt aus dem Bereich 45° - 55° (einschließlich der Grenzen) auf. Der erste Winkel kann beispielsweise 46°, 47°, 48°, 49°, 50°, 51°, 52° oder 53° betragen.

Der Fluidanschluss kann so ausgebildet sein, dass er eine Flasche (bzw. einen Behälter) mit dem Fluid (z.B. eine Medikamentenflasche) haltern kann. Der Fluidanschluss ist bevorzugt so ausgebildet, dass die Achse der Flasche senkrecht zur Ebene ist, in der die ebene Stirnfläche des Abgabeendes liegt. Wenn beim bestimmungsgemäßen Gebrauch des Tropfenapplikators die ebene Stirnfläche parallel zur Erdoberfläche ausgerichtet ist, ist die Achse der Flasche parallel zur Vertikalen, so dass leicht eine maximale Entleerung der Flasche erreicht werden kann.

Mit dem Flächeninhalt der ebenen Stirnfläche des Abgabeendes kann das Tropfenvolumen festgelegt werden, bei dem sich der Tropfen durch die Erdanziehung vom Abgabeende der Austrittsdüse löst und abfällt. Je größer die Fläche der oberen Stirnfläche, desto größer sind die Adhäsionskräfte, die durch die Gravitation überwunden werden müssen. Deshalb kann vorteilhaft mittels des festgelegten Flächeninhalts das gewünschte Tropfenvolumen eingestellt werden. Für z.B. ein mittleres Tropfenvolumen von 0,028 ml ergibt sich ein Flächeninhalt der Stirnfläche von 2,9 mm².

Der rohrförmige Endabschnitt der Austrittsdüse steht bevorzugt über das Gehäuse vor, so dass leicht eine gewünschte Positionierung der ebenen Stirnfläche (bevorzugt parallel zur Horizontalen) bei der Tropfenabgabe erreicht werden kann.

Da die ebene Stirnfläche des Abgabeendes zur Unterseite hin weist und einen ersten Winkel von kleiner als 90° mit der ersten Längsrichtung einschließt, kann zusätzlich ein Zurücklaufen des Tropfens (oder Teile davon) an der Außenseite des rohrförmigen Endabschnitts bei der Tropfenabgabe verhindert werden, was zur hohen Dosiergenauigkeit beiträgt.

Somit kann mit der erfindungsgemäßen Vorrichtung gewährleistet werden, dass sich pro Betätigung des Betätigungselementes nur ein einzelner Tropfen eines definierten Volumens löst. Dadurch kann der Verlust von Impfstoff reduziert werden.

Außerdem kann durch die räumliche Trennung des Fluidanschlusses (an dem z.B. ein Reservoir oder eine Flasche mit dem gewünschten Fluid, z.B. ein flüssige Medikament, angeordnet werden kann) und den Bereichen des Gehäuses, an dem der Tropfenapplikator beim bestimmungsgemäßen Gebrauch gehalten wird, eine unerwünschte Erwärmung des Fluids (z.B. des Medikaments) durch Körperwärme ausgeschlossen werden.

Die Unterseite des Gehäuses kann sich entlang einer zweiten Längsrichtung erstrecken, wobei, im gedachten Längsschnitt des rohrförmigen Endabschnitts, die erste Längsrichtung mit der zweiten Längsrichtung einen zweiten Winkel einschließt, der zur Unterseite hin offen und kleiner als 180° ist. Insbesondere kann der zweite Winkel kleiner oder gleich 175°, 170°, 165°, 160°, 155°, 150° oder 145° sowie größer oder gleich 100°, 105°, 110°, 115°, 120°, 125°, 130°, 135°, 140°, 145°, 150°, 155°, 160°, 165° oder 170° sein. Bevorzugt liegt der zweite Winkel in einem Bereich von 175° - 145° (einschließlich der Grenzen) und besonders bevorzugt 170° - 150° (einschließlich der Grenzen). Der zweite Winkel kann insbesondere 170°, 169°, 168°, 167°, 166°, 165°, 164°, 163°, 162°, 161°, 160°, 159°, 158°, 157°, 156° oder 155° betragen.

Das Gehäuse kann einen den Fluidanschluss aufweisenden Anschlussabschnitt umfassen, der von der Oberseite des Gehäuses vorsteht. Insbesondere kann er schräg von der Oberseite des Gehäuses vorstehen. Der Anschlussabschnitt kann sich entlang einer dritten Längsrichtung erstrecken, wobei, im gedachten Längsschnitt des rohrförmigen Endabschnitts, die dritte Längsrichtung mit der ebenen Stirnfläche einen dritten Winkel aus einem Bereich von 80° - 100° (einschließlich der Grenzen) und bevorzugt aus einem Bereich von 85° - 95° (einschließlich der Grenzen) einschließt. Besonders bevorzugt beträgt der dritte Winkel 85°, 86°, 87°, 88°, 89°, 90°, 91°, 92°, 93°, 94° oder 95° und besonders bevorzugt beträgt er 90°.

Die Fluidverbindung kann einen elastischen Schlauch aufweisen, gegen den das Betätigungselement bei Betätigung gedrückt wird, um den Schlauchquerschnitt zu verringern und so die gewünschte Fluidmenge für einen abzugebenden Tropfen zur Austrittsdüse zu fördern.

Bei Betätigung des Betätigungselementes kann somit dieses gegen eine Anlagestelle des elastischen Schlauches gedrückt werden, wobei stromaufwärts und stromabwärts der Anlagestelle jeweils ein Rückschlagventil im elastischen Schlauch angeordnet sein kann. Die beiden Rückschlagventile können insbesondere so ausgelegt sein, dass ab einem vorbestimmten Überdruck im elastischen Schlauch zwischen den beiden Rückschlagventilen das stromabwärts angeordnete Rückschlagventil öffnet und ab einem vorbestimmten Unterdruck im elastischen Schlauch zwischen den beiden Rückschlagventilen das stromaufwärts angeordnete Rückschlagventil öffnet.

Damit kann auf mechanisch sehr einfache und damit auch kostengünstige Art und Weise das Fördern der gewünschten Fluidmenge zur Abgabe eines Tropfens zur Austrittsdüse realisiert werden.

Das Betätigungselement kann einen im Gehäuse verschiebbar gelagerten stangenförmigen Abschnitt aufweisen, dessen dem elastischen Schlauch zugewandtes Ende bei Betätigung des Betätigungselementes gegen den elastischen Schlauch gedrückt wird.

Das Betätigungselement kann ferner einen über das Gehäuse (bevorzugt über die Oberseite des Gehäuses bzw. die Oberseite eines entsprechenden Halteabschnittes des Gehäuses) vorstehenden Endabschnitt aufweisen, auf den eine Kraft ausgeübt werden kann, um das Betätigungselement gegen den Schlauch zu drücken.

Die Rückstellkraft des elastischen Schlauches kann so groß sein, dass nur die Gewichtskraft des Betätigungselementes zu gering ist, um Fluid zur Austrittsdüse zu fördern.

Über die Länge des Betätigungselementes kann somit bestimmt werden, wie viel Fluidvolumen pro Betätigung gefördert wird, wobei dies auf den Flächeninhalt der Stirnseite abgestimmt ist, um die gewünschte Tropfengröße wiederholbar zu erzeugen.

Wenn die Länge des Betätigungselementes zu kurz, wird nicht genügend Fluid gefördert und der Tropfen löst sich nicht von der Stirnfläche. Ist die Länge des Betätigungselementes aber zu lang, wird zu viel Fluid gefördert, was zur Folge haben kann, dass sich nach dem Lösen des Tropfens ein weiterer Teiltropfen bildet, der in Addition mit dem Fluid aufgrund einer darauffolgenden Betätigung des Betätigungselementes zu einem Doppeltropfen führen kann.

Das Gehäuse kann im vorderen Bereich zwei voneinander wegweisende Griffmulden (oder zwei voneinander wegweisende Fingermulden) für Daumen einerseits und Mittelfinger und Ringfinger andererseits eines Benutzers aufweisen, um das Gehäuse in der sogenannten Drei-Punkt-Haltung halten zu können. Dabei liegt die Unterseite des Gehäuses auf der Einbuchtung der Handrückenseite zwischen Daumen und Zeigefinger, wobei Daumen und Mittelfinger (oder Ringfinger) an den Fingermulden anliegen. Das Betätigungselement ist bevorzugt so angeordnet, dass bei der Drei-Punkt-Haltung mit dem Zeigefinger das Betätigungselement gegen den elastischen Schlauch gedrückt werden kann. Somit kann die erfindungsgemäße Vorrichtung in Art eines Stiftes mit einer Hand gehalten und bedient werden. Die Tropfenabgabe ist somit einhändig möglich.

Die Griffmulden können insbesondere so am Gehäuse angeordnet sein, dass die erfindungsgemäße Vorrichtung gleichermaßen für Rechtshänder wie auch für Linkshänder geeignet ist.

Das Gehäuse, das Betätigungselement und die Austrittsdüse können aus Kunststoff hergestellt sein. Für das Gehäuse und das Betätigungselement kann z.B. PC und/oder ABS verwendet werden. Für die Austrittsdüse kann z.B. POM verwendet werden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbeispiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine perspektivische Ansicht eines Ausführungsbeispiels der Vorrichtung 1 zur tropfenweisen Abgabe eines Fluids, bei der die linke Seite des Gehäuses 2 sichtbar ist;
- Fig. 2: eine perspektivische Darstellung der Vorrichtung 1 von Figur 1, wobei die rechte Seite des Gehäuses 2 sichtbar ist;
- Fig. 3: eine Ansicht der Unterseite 10 des Gehäuses 2 der Vorrichtung 1 gemäß Figur 1;
- Fig. 4: ein Längsschnitt der Vorrichtung 1 gemäß Figur 1;
- Fig. 5: eine vergrößerte Darstellung des Details A von Figur 3;
- Fig. 6: eine vergrößerte Darstellung des Details B von Figur 4;
- Fig. 7: eine Darstellung zur Erläuterung der unterschiedlichen Längsrichtungen R1 - R3, deren Orientierungen sowie die Orientierungen relativ zur Stirnfläche 13;
- Fig. 8: eine perspektivische Darstellung der Vorrichtung 1 gemäß Figur 1, wobei der Aufnahmeanschluss 16 einen Behälter 17 haltert;
- Fig. 9: eine Längsschnittansicht der Vorrichtung 1 mit dem Behälter 17 gemäß Figur 8, und
- Fig. 10: die Längsschnittansicht gemäß Figur 9 mit eingezeichnetem Fluid FL.

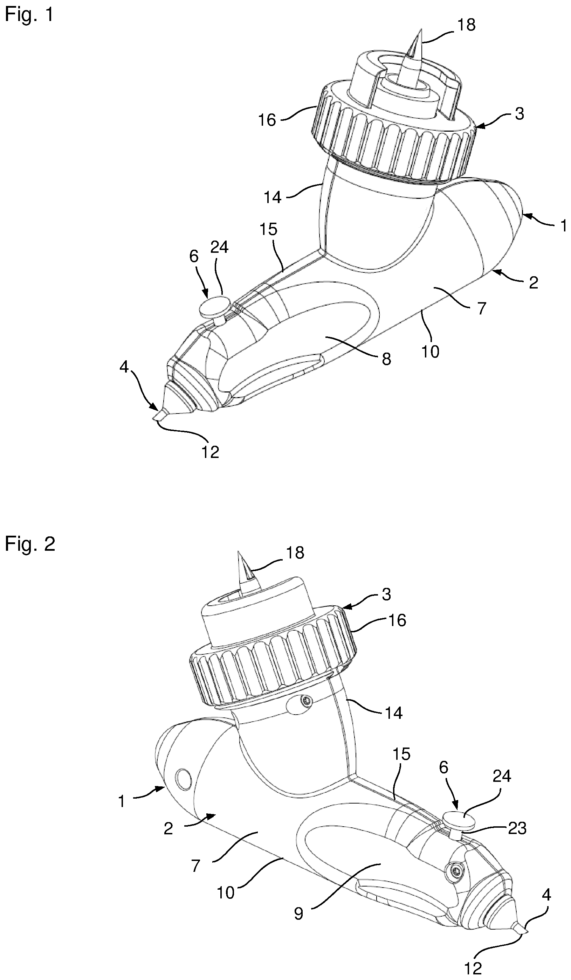

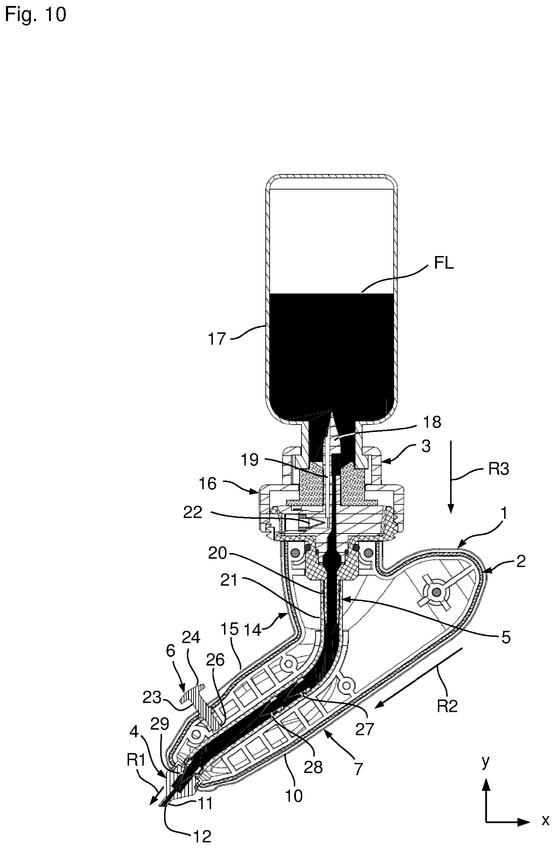

Bei dem in Figuren 1 bis 10 gezeigten Ausführungsbeispiel weist die erfindungsgemäße Vorrichtung 1 zur tropfenweisen Abgabe eines Fluids (die auch als Tropfenapplikator 1 bezeichnet werden kann) ein Gehäuse 2 mit einem Fluidanschluss 3, über den das abzugebende Fluid zuführbar ist, einer Austrittsdüse 4, über die das Fluid tropfenweise abgegeben werden soll, einer Fluidverbindung 5, die den Fluidanschluss 3 mit der Austrittsdüse 4 verbindet, und einem Betätigungselement 6 auf.

Das Gehäuse 2 umfasst einen sich länglich erstreckenden Halteabschnitt 7 mit zwei seitlichen Fingermulden 8, 9 im vorderen Bereich des Halteabschnitts 7, die für Daumen und Mittelfinger (oder Ringfinger) eines Benutzers ausgebildet sind. Der Halteabschnitt 7 umfasst eine Unterseite 10, die sich entlang einer Längsrichtung R2 erstreckt. Beim bestimmungsgemäßen Gebrauch der Vorrichtung 1 hält der Benutzer die Vorrichtung 1 wie einen Stift in der sogenannten Drei-Punkt-Haltung. Dabei liegt die Unterseite 10 des Gehäuses 2 auf der Einbuchtung der Handrückenseite zwischen Daumen und Zeigefinger, wobei Daumen und Mittelfinger (oder Ringfinger) an den Fingermulden 8, 9 anliegen. Mit dem Zeigefinger kann dann das Betätigungselement 6 nach unten gedrückt werden, um, wie nachfolgend noch im Detail beschrieben wird, den gewünschten Tropfen des Fluids über die Austrittsdüse 4 abzugeben.

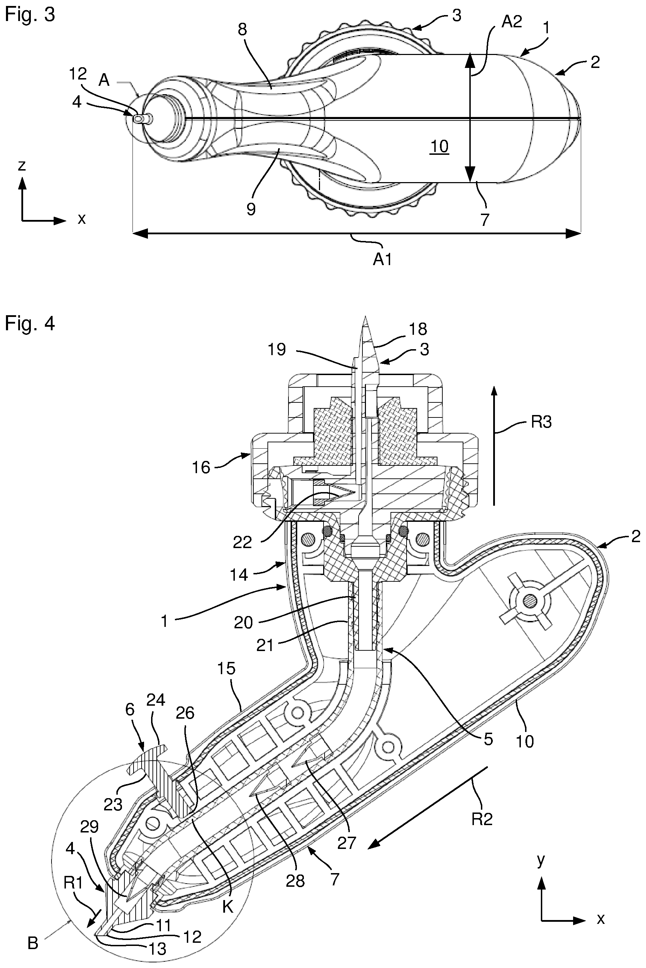

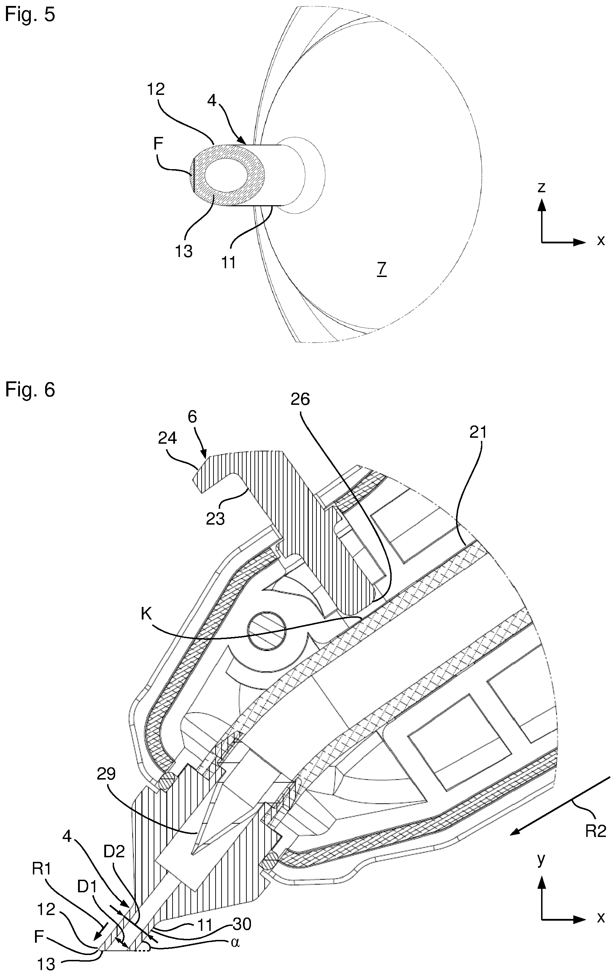

Die Austrittsdüse 4 umfasst einen sich geradlinig entlang einer Längsrichtung R1 erstreckenden rohrförmigen Endabschnitt 11 mit einem abgeschrägten Abgabeende 12, so dass eine ebene ringförmige Stirnfläche 13 ausgebildet ist, die zur Unterseite 10 hin weist und einen Winkel α von kleiner als 90° mit der Längsrichtung R1 einschließt (siehe insbesondere Figuren 4, 6 und 7). Der Winkel a ist zur Unterseite 10 hin offen. Beim gezeigten Ausführungsbeispiel ist der Winkel α gleich 52°. An der vom Gehäuse 2 wegweisenden Seite des abgeschrägten Abgabeendes 12 kann eine Fase F (Fig. 5) ausgebildet sein. Jedoch ist es auch möglich, keine Fase vorzusehen.

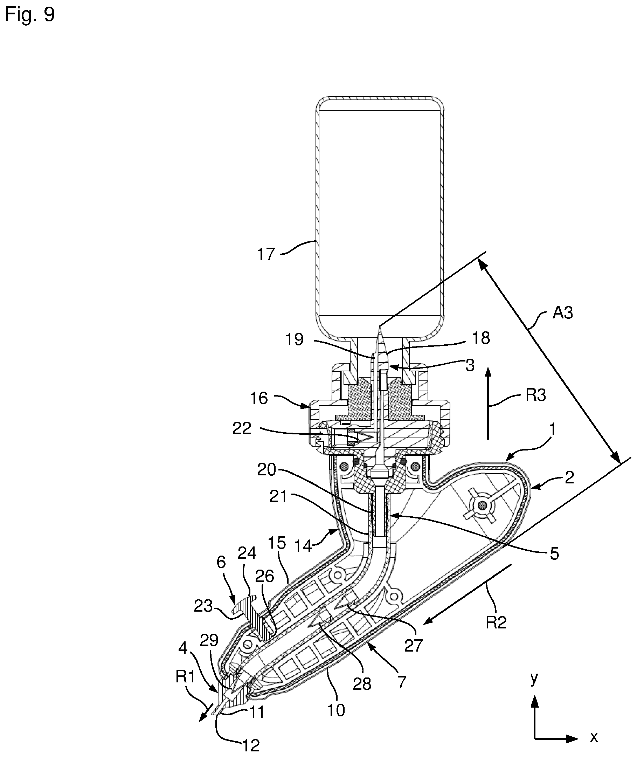

Der rohrförmige Endabschnitt 11 kann einen Innendurchmesser D1 von 1,1 mm und einen Außendurchmesser D2 von 1,9 mm aufweisen (Fig. 6). Der Tropfenapplikator 1 kann eine Höhe A3 von 71 mm (Figur 9) und eine Länge A1 von 111 mm (Figur 3) aufweisen. Die Breite A2 des Halteabschnitts 7 kann 26 mm betragen (Figur 3).

Wie z.B. in Figuren 1, 2, 4 und 6 angedeutet ist, ist bei dem bestimmungsgemäßen Gebrauch des Tropfenapplikators 1 aufgrund der beschriebenen Drei-Punkt-Haltung das Gehäuse 2 gegenüber der Horizontalen (hier durch die x-Richtung angedeutet) geneigt, so dass bei diesem ergonomischen Halten des Tropfenapplikators 1 die Stirnfläche 13 leicht parallel zur Horizontalen positioniert werden kann. Dies führt, wie nachfolgend noch beschrieben wird, in vorteilhafter Weise unter anderem dazu, dass wiederholbar ein Tropfen mit gleicher Fluidmenge abgebbar ist.

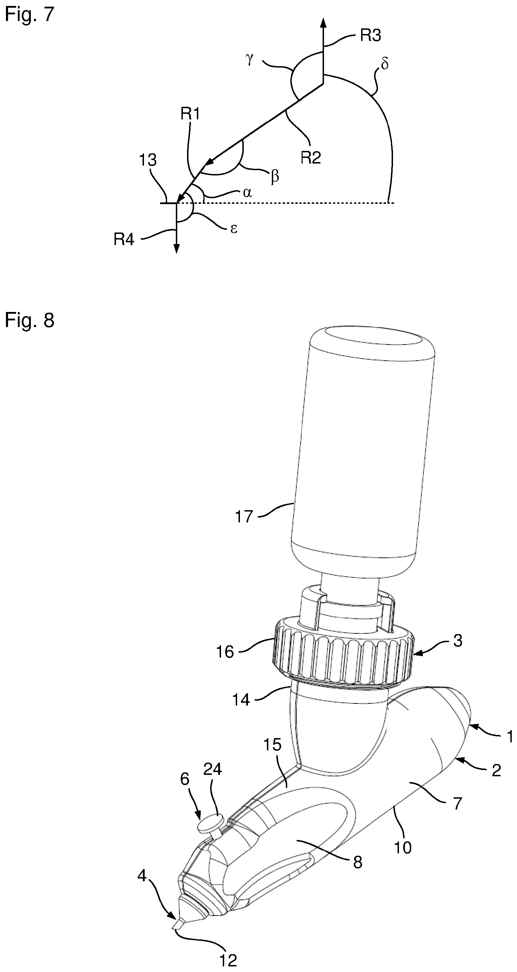

Das Gehäuse 2 weist ferner einen Anschlussabschnitt 14 auf, der von einer Oberseite 15 des Halteabschnitts 7 schräg vorsteht. Wie beispielsweise aus Figur 4 ersichtlich ist, erstreckt sich der Anschlussabschnitt 14 entlang einer Längsrichtung R3. Die Ausrichtung dieser Längsrichtung R3 ist bevorzugt so gewählt, dass sie mit der Vertikalen (hier die y-Richtung in Fig. 4) zusammenfällt, wenn die Stirnfläche 13 parallel zur Horizontalen ausgerichtet ist. Dies kann dadurch leicht erreicht werden, dass im Längsschnitt gemäß Figur 4, wie in Figur 7 gezeigt ist, der Winkel δ, den die Stirnfläche 13 und die Richtung R3 einschließen, im Bereich von 85°- 95° liegt. Bevorzugt beträgt der Winkel δ 90°.

Der Anschlussabschnitt 14 kann in der Mitte bis zum hinteren Bereich des Halteabschnitts 7 positioniert und ausgebildet sein.

Der Anschlussabschnitt 14 weist eine Aufnahme 16 für einen Behälter 17 (wie beispielsweise in Fig. 9 - 10 gezeigt ist) mit abzugebendem Fluid FL auf, wobei ein Einstichdorn 18 der Aufnahme 16 in den Behälter 17 hineinsteht. Im Einstichdorn 18 ist ein Kanal 19 ausgebildet, der sich weiter durch die Aufnahme 16 bis zu einem Kanalendabschnitt 20 innerhalber des Gehäuses 2 erstreckt. Auf den Kanalendabschnitt 20 ist ein elastischer Schlauch 21 aufgeschoben, der den Kanalendabschnitt 20 mit der Austrittsdüse 4 verbindet. In dieser Art und Weise ist die Fluidverbindung 5 zwischen dem Fluidanschluss 3 und der Austrittsdüse 4 verwirklicht.

Im Anschlussabschnitt 14 ist ein mit dem Kanal 19 verbundenes Rückschlagventil 22 vorgesehen, über das Luft dem Kanal 19 zugeführt werden kann, um einen Unterdruck im Behälter 17 bei Abgabe des Fluids FL zu verhindern.

Im Längsschnitt gemäß Figuren 4 und 6 gesehen liegt somit der beschriebene Winkel α vor. Man kann auch sagen, dass in diesem Längsschnitt gemäß Figuren 4 und 6 gesehen, die ebene Stirnfläche 13 und die zur Unterseite 10 weisende Außenfläche 30 V-förmig angeordnet sind, wobei das V von der Unterseite 10 weg bzw. zur Oberseite 15 hin offen ist.

Wie insbesondere Figuren 4 und 6 entnommen werden kann, ist im vorderen Bereich der Oberseite 15 das Betätigungselement 6 in Form eines verschiebbar gelagerten stangenförmigen Abschnitts 23 ausgebildet, der an seinem von der Oberseite 15 wegweisenden Ende einen Endabschnitt 24 (hier als Anlageplatz 24) aufweist und mit seinem entgegengesetzten Ende 26 zu einer Anlagestelle K am Schlauch 21 weist. Zwischen der Anlagestelle K und der Aufnahme 16 (und somit stromaufwärts) sind innerhalb des Schlauches 21 zwei Rückschlagventile 28 und 29 und zwischen der Anlagestelle K und der Austrittsdüse 4 (und somit stromabwärts) ist ein weiteres Rückschlagventil 29 angeordnet.

Wie beispielsweise gut in Figuren 4, 6 und 7 zu erkennen ist, ist der rohrförmige Endabschnitt 11 zur Längsrichtung R2, entlang sich der elastische Schlauch 21 (und somit der Fluidkanal 5) im Bereich des Betätigungselementes 6 erstreckt, geneigt. Im hier beschriebenen Ausführungsbeispiel schließen die beiden Längsrichtungen R1 und R2 einen zur Unterseite 10 hin offenen Winkel β ein, der hier 163° beträgt. Um zu den oben angegebenen Werten für den Winkel δ zu gelangen, schließen die Längsrichtungen R2 und R3 einen zur Oberseite hin offenen Winkel γ ein, der hier 125° beträgt. Dies führt zu einem Winkel ε von 142° zwischen der Längsrichtung R1 und einer Senkrechten R4 auf die Stirnfläche 13. Wie bereits angegeben, wird bei dem bestimmungsgemäßen Gebrauch des Tropfenapplikators 1 dieser so gehalten, dass die ebene Stirnfläche 13 parallel zur Horizontalen positioniert ist, so dass die Senkrechte R4 in diesem Fall der Richtung entspricht, entlang der der jeweilige Tropfen abgegeben wird.

Das Gehäuse 2 kann aus PC/ABS (Polycarbonat/Acrylnitril-Butadien-Styrol) hergestellt sein. Die Austrittsdüse 4 kann aus POM (Polyoxymethylen) hergestellt sein.

Wird nun das Betätigungselement 6 durch einen Benutzer, der die Vorrichtung 1 in der Drei-Punkt-Haltung hält, mit dem Zeigefinger gegen den elastischen Schlauch 21 gedrückt, wird ein Überdruck im Schlauch 21 zwischen den Rückschlagventilen 28 und 29 erzeugt, der dazu führt, dass sich das Rückschlagventil 29 öffnet (wohingegen die Rückschlagventile 27 und 28 geschlossen bleiben) und die gewünschte Fluidmenge über das Abgabeende 12 als Tropfen abgegeben wird. Wird das Betätigungselement 6 nicht mehr mit Druck (z.B. mittels des Zeigefingers) beaufschlagt, wird es aufgrund der Rückstellkraft des elastischen Schlauches 21 zur Oberseite 15 hin bewegt, so dass das Rückschlagventil 29 schließt und der dadurch erzeugte Unterdruck zwischen dem Rückschlagventil 29 und dem Rückschlagventil 28 dazu führt, dass die Rückschlagventile 27 und 28 öffnen, so dass Fluid FL aus dem Behälter 17 in dem Bereich zwischen den Rückschlagventilen 28 und 29 nachfließen kann.

Wenn ein Behälter 17 mit Fluid FL mit dem Aufnahmeanschluss 16 verbunden wird und in der Fluidverbindung 5 von der Aufnahme 16 bis zur Austrittsdüse 4 noch kein Fluid FL vorhanden oder die Fluidverbindung 5 nicht vollständig mit Fluid FL gefüllt ist, wird zur Befüllung bzw. Auffüllung das Betätigungselement 6 ein oder mehrmals betätigt, bis Fluid FL am Abgabeende 12 der Austrittsdüse 4 sichtbar ist. Damit ist die Vorrichtung 1 einsatzbereit, und jede weitere Betätigung des Betätigungselements 6 führt zur Abgabe des gewünschten Fluidtropfens.

Das Vorsehen von zwei Rückschlagventilen 27 und 28 ist nicht zwingend. Es reicht auch ein einzelnes Rückschlagventil. Dies hängt von der Auslegung der Vorrichtung 1 und Eigenschaften der Rückschlagventile 27 - 29 ab. Im hier vorliegenden Ausführungsbeispiel wurden Standardrückschlagventile verwendet, so dass zwischen dem Anschlussabschnitt 14 und dem Betätigungselement 6 die zwei Rückschlagventile 27 und 28 anzuordnen waren.

Der Halteabschnitt 7 kann so ausgebildet sein, dass der Tropfenapplikator 1 sowohl nur mit der rechten Hand als auch nur mit der linken Hand bedient werden kann. Wenn er mit der linken Hand gehalten wird, liegt der Daumen an der Fingermulde 9 und der Mittelfinger (oder Ringfinger) an der Fingermulde 8 an.

## Patentansprüche

1. Vorrichtung zur tropfenweise Abgabe eines Fluids,
wobei die Vorrichtung (1) ein Gehäuse (2) mit
einem Fluidanschluss (3), über den das abzugebende Fluid (F) zuführbar ist,
eine Austrittsdüse (4), über die das Fluid tropfenweise abgebbar ist,
einer Fluidverbindung (5), die den Fluidanschluss (3) mit der Austrittsdüse (4) verbindet,
einer Unterseite (10) und
einem Betätigungselement (6) aufweist,
wobei bei Betätigung des Betätigungselements (6) eine vorbestimmte Fluidmenge zur Austrittsdüse (4) hin gefördert wird,
wobei die Austrittsdüse (4) einen sich geradlinig entlang einer ersten Längsrichtung (R1) erstreckenden rohrförmigen Endabschnitt (11) mit einem abgeschrägten Abgabeende (12), das eine ebene Stirnfläche (13) umfasst, aufweist,
und wobei, in einem gedachten Längsschnitt des rohrförmigen Endabschnitts (11), die ebene Stirnfläche (13) zur Unterseite (10) hin weist und mit der ersten Längsrichtung (R1) einen ersten Winkel (α) einschließt, der kleiner als 90° ist.

2. Vorrichtung nach Anspruch 1, bei der
sich die Unterseite (10) des Gehäuses (2) entlang einer zweiten Längsrichtung (R2) erstreckt, und wobei, im gedachten Längsschnitt des rohrförmigen Endabschnitts (11), die erste Längsrichtung (R1) mit der zweiten Längsrichtung (R2) einen zweiten Winkel (β) einschließt, der zur Unterseite (10) hin offen und kleiner als 180° ist.

3. Vorrichtung nach Anspruch 1 oder 2, bei der
das Gehäuse (2) einen den Fluidanschluss (5) aufweisenden Anschlussabschnitt (14) umfasst, der von der Oberseite (15) des Gehäuses (2) vorsteht und sich entlang einer dritten Längsrichtung (R3) erstreckt,
wobei, im gedachten Längsschnitt des rohrförmigen Endabschnitts (11), die dritte Längsrichtung (R3) mit der ebenen Stirnfläche (13) einen dritten Winkel (δ) aus einem Bereich von 85° bis 95° einschließt.

4. Vorrichtung nach Anspruch 3, bei der
der Anschlussabschnitt (14) von der Oberseite (15) des Gehäuses (2) schräg vorsteht.

5. Vorrichtung nach einem der obigen Ansprüche, bei der
die Fluidverbindung (5) einen elastischen Schlauch (21) aufweist, gegen den das Betätigungselement (6) bei Betätigung gedrückt wird, um den Schlauchquerschnitt zu verringern und so die gewünschte Fluidmenge zur Austrittsdüse (4) zu fördern.

6. Vorrichtung nach Anspruch 5, bei der
das Betätigungselement (6) bei Betätigung gegen eine Anlagestelle (K) des elastischen Schlauches (21) gedrückt wird,
wobei stromaufwärts und stromabwärts der Anlagestelle (K) jeweils ein Rückschlagventil (27, 28; 29) im elastischen Schlauch (21) angeordnet ist,
wobei ab einem vorbestimmten Überdruck zwischen den beiden Rückschlagventilen (27, 28; 29) das stromabwärts angeordnete Rückschlagventil (29) und ab einem vorbestimmten Unterdruck im elastischen Schlauch zwischen den beiden Rückschlagventilen (27, 28; 29) das stromaufwärts angeordnete Rückschlagventil (27, 28) öffnet.

7. Vorrichtung nach Anspruch 5 oder 6, bei der
das Betätigungselement (6) einen im Gehäuse (2) verschiebbar gelagerten stangenförmigen Abschnitt (23) aufweist, dessen dem elastischen Schlauch (21) zugewandtes Ende (26) bei Betätigung des Betätigungselementes (6) gegen den elastischen Schlauch (21) gedrückt wird.

8. Vorrichtung nach einem der Ansprüche 5 bis 7, bei der
das Betätigungselement (6) einen über das Gehäuse (2) vorstehenden Endabschnitt (24) aufweist, auf den eine Kraft ausgeübt werden kann, um das Betätigungselement (6) gegen den elastischen Schlauch (21) zu drücken.

9. Vorrichtung nach einem der Ansprüche 5 bis 8, bei der
die Rückstellkraft des elastischen Schlauches (21) so groß ist, dass nur die Gewichtskraft des Betätigungselementes (6) zu gering ist, um Fluid zur Austrittsdüse (4) zu fördern.

10. Vorrichtung nach einem der obigen Ansprüche, bei der
das Gehäuse (2) im vorderen Bereich zwei voneinander wegweisende Griffmulden (8, 9) für Daumen einerseits und Mittelfinger oder Ringfinger andererseits eines Benutzers aufweist, um das Gehäuse (2) in der Drei-Punkt-Haltung halten zu können.

11. Vorrichtung nach einem der obigen Ansprüche, bei der
der erste Winkel (α) im Bereich von 40° - 60° liegt.

12. Vorrichtung nach einem der obigen Ansprüche, bei der der rohrförmige Endabschnitt (11) über das Gehäuse (2) vorsteht.
